# EUROPEAN PATENT APPLICATION

(11) **EP 2 216 020 A1**
(43) Date of publication of application: **11.08.2010**
(21) Application number: 09001641.1
(22) Date of filing: 05.02.2009
(51) Int. Cl.: A61K 31/165, A61K 9/26

(54) **Moisture-activated granulation process**

(71) Applicant: KRKA, tovarna zdravil, d.d., Novo mesto, 8501 Novo mesto (SI)
(72) Inventor: Stukelj, Mitja, 8000 Novo mesto (SI); Vrecer, Franc, 8351 Straza pri Novem mestu (SI); Skrabanja, Vida, 8000 Novo mesto (SI)
(74) Representative: UEXKÜLL & STOLBERG

(57) **Abstract**

The present invention relates to a moisture-activated granulation process for the manufacture of a pharmaceutical dosage form containing a moisture-sensitive active substance such as aliskiren, said process comprising:
a) mixing the active ingredient with one or more dry excipients such as diluents, surfactants, binders, lubricants, disintegrators, granulation aids, buffering/alkalizing agents and antioxidants in solid form,;
b) contacting the mixture with a granulation liquid containing water and optionally one or more excipients such as binders, buffering/alkalizing agents, surfactants and antioxidants to form a granulate, wherein the ratio of the total amount of the excipients of the granulate to water is less than 100:30, based on weight;
c) further processing the mixture including the addition of further dry excipients such as diluents, disintegrants, glidants and lubricants to obtain the pharmaceutical dosage form.

The invention also relates to a dosage form obtainable by this process and its use in the treatment of medical conditions.

## Description

The present invention relates to a moisture-activated granulation process for manufacturing pharmaceutical compositions, in particular solid dosage forms of active substances which are prone to chemical degradation and/or physical phase transitions upon contact with water or aqueous liquids such as those used during conventional wet granulation processes. The present invention also relates to the pharmaceutical formulations and dosage forms obtained from the process according to the invention and their use as medicaments.

### Background of the Invention

Numerous active substances are sensitive to the presence of moisture. This can pose significant problems in the manufacture of pharmaceutical formulations and dosage forms containing such active substances. Moisture may stem from the excipients used in the formulation or from the manufacturing process, e.g. aqueous granulation.

The presence of moisture is in particular undesirable if the active substance is prone to chemical degradation and/or physical phase transitions into an undesired crystalline and/or amorphous form (polymorphism) when being in contact with water or water-containing solutions. Examples for such active substances are aliskiren, in particular aliskiren hemifumarate, ACE inhibitors selected from lisinopril, ramipril, enalapril, teophiline, valsartan, orlistat, desloratidine, solifenacin and its salts such as the maleate, malonate, hydrogensulphate, succinate and citrate, donepezil and its salts, benzimidazole proton pump inhibitors selected from omeprazole, esomeprazole, rabeprazole, pantoprazole, lansoprazole and their salts such as sodium and magnesium salts, inhibitors of HGMCo reductase selected from rosuvastatin, atorvastatin and its salts, in particular atorvastatin Ca, fluvastatin and its salts, platelet aggregation inhibitors selected from clopidrogel, in particular clopidogrel salts with HCl and H2SO4, and prasugrel. Said active substances are in particular known to form chemical degradation products when moisture is present in the pharmaceutical formulation containing them.

Other active substances such as teophiline, pantoprazole, aliskiren and others are in particular known to undergo physical transformation into undesired crystalline and/or amorphous forms in the course of manufacturing processes including steps wherein the active substance is contacted with moisture and subjected to subsequent drying. One specific example for a manufacturing process where this problem may occur is wet granulation, in particular wet granulation using water or aqueous liquids such as aqueous solutions or dispersions as the granulation liquid. This type of wet granulation is still very common in the manufacturing of solid dosage forms such as tablets and capsules.

For example, it is known that aliskiren hemifumarate is converted to an amorphous form when processed by using aqueous wet granulation using water and/or aqueous binder solutions.

The prior art has attempted to solve the above problems by using alternative granulation methods and liquids. For example, WO 2005/089729 and US 2006/0018960 Al suggest a process for preparing a formulation based on aliskiren as the active substance using wet granulation of aliskiren using a mixture of organic solvents or a binder solution in organic solvents. Thus, the use of organic solvents is an essential feature of the manufacturing process disclosed in these two prior art documents. The drawback of such a process is that residual solvents and impurities in the product of such solvent-based granulation methods may render the resulting formulation unstable and that drying steps are needed. The prior art discussed above also contains a very general reference to basically any other method of granulation, see. e.g. paragraph [0090] of US 2006/0018960 Al. However, this does not change the essential teaching of the invention of US 2006/0018960 Al and WO 2005/089729 that using a granulation liquid, i.e. a mixture of organic solvents, is mandatory to achieve a satisfactory result, i.e. to avoid contact with water.

EP-A-l 972 335 and WO-A-2008/116601 relate to a process for the manufacture of a pharmaceutical formulation comprising (a) granulating aliskiren or a pharmaceutical salt thereof alone or in admixture with one or more excipients selected from binders, fillers, disintegrants, wetting agents and/or lubricants at elevated temperature and in the essential absence of solvents such as those used in the prior art discussed in the preceding paragraph; and (b) optionally formulating the granulate obtained in step (a) into tablets, film-coated tablets, pills, lozenges, sachets, soft or hard gelatine capsules.

However, a need still exists for providing methods for manufacturing formulations containing aliskiren or other moisture-sensitive active substances for in particular effective oral delivery, which methods take account of the demanding charactistics of the active substance, in particular in relation to their processing, and which provide pharmaceutical formulations having an appropriate disintegration time and/or appropriate solubility and/or dissolution profile of the active principle.

The inventors of the present application have surprisingly found that a certain type of aqueous granulation process can be used for the above type of active substances, i.e. active substances prone to chemical degradation and/or physical phase transformations (polymorphism) upon contact with water or aqueous liquids, when certain limitations concerning the amount and contacting time of the granulation liquid are observed. In particular, the inventors of the present application have surprisingly succeeded in manufacturing dosage forms having good stability and bioavailability, said dosage forms containing moisture sensitive active substances such as aliskiren and its pharmaceutically acceptable salts, by using a moisture-activated granulation process.

More specifically, in accordance with the present invention there is provided a moisture-activated granulation process for the manufacture of a pharmaceutical dosage form containing at least one moisture-sensitive active substance selected from the group consisting of aliskiren and its pharmaceutically acceptable salts, said process comprising:
a) mixing the active ingredient(s) with one or more dry excipients in solid form, said excipient being selected in particular from the group consisting of diluents, surfactants, binders, lubricants, disintegrators, granulation aids, buffering/alkalizing agents and antioxidants;
b) contacting the mixture with a granulation liquid containing water and optionally one or more excipients to form a granulate, said excipient being selected in particular from the group consisting of binders, buffering/alkalizing agents, surfactants and antioxidants,
   wherein the ratio of the total amount of the excipients of the granulate to water is less than 100:30, based on weight; and
c) further processing the mixture including the addition of dry excipients to obtain the pharmaceutical dosage form, said excipients being selected in particular from the group consisting of diluents, disintegrants, glidants and lubricants.

The present invention also provides a pharmaceutical dosage form containing a moisture-sensitive active substance said formulation being obtainable by a process as defined above.

The present invention also provides a pharmaceutical dosage form containing a moisture-sensitive active substance as defined above for the treatment of hypertension, congestive heart failure, angina, myocardial infarction, artherosclerosis, diabetic nephropathy, diabetic cardiac myopathy, renal insufficiency, peripheral vascular disease, left ventricular hypertrophy, cognitive dysfunction, stroke, headache and chronic heart failure.

Further preferred embodiments of the present invention are disclosed in the dependent claims attached to this description.

In accordance with the present invention, the term "dry" means that the water content of the excipients which are mixed with the one or more active ingredient in step (a) is less than 2 wt-%, preferably less than 1.5 wt-%, based on the total weight of the excipient mixed with the active ingredient and determined by the loss on drying method according to Ph. Eur. Chapter 2.2.32 (determined on 1.000g by drying in an oven at 105°C for 3 hours).

Preferably, the ratio of the total amount of the excipients used in steps (a) and (b) to water in the granulation liquid is less than 100:20, in particular less than 100:10, based on weight.

In case excipients such as binders, buffering/alkalizing agents, surfactants or antioxidants are dissolved or dispersed in the water of the granulation liquid, they are taken into account in the calculation of the above ratios as solid, dry excipients.

In general, the excipients and the active ingredient(s) which are mixed in step (a) are present in the form of finely divided solid particles, e.g. as powders. Usually, the particles have a size in the range of micrometers, e.g. 0.1 and up to 300 µm. Preferably, the dry components are mixed in the form of powders.

In the following description, any reference to the term active substance is intended to include the pharmaceutically acceptable salts thereof. For example, when referring to "aliskiren" this includes the pharmaceutically acceptable salts thereof such as in particular aliskiren hemifumarate. The same principle applies to the use of the names of other specific actives. Specifically, the following active substances may be processed in accordance with the present invention: aliskiren, in particular aliskiren hemifumarate, ACE inhibitors selected from lisinopril, ramipril, enalapril, teophiline, valsartan, orlistat, desloratidine, solifenacin and its salts such as the maleate, malonate, hydrogensulphate, succinate and citrate, donepezil and its salts, benzimidazole proton pump inhibitors selected from omeprazole, esomeprazole, rabeprazole, pantoprazole, lansoprazole and their salts such as sodium and magnesium salts, inhibitors of HGMCo reductase selected from rosuvastatin, atorvastatin and its salts, in particular atorvastatin Ca, fluvastatin and its salts, platelet aggregation inhibitors selected from clopidrogel, in particular clopidogrel salts with HCl and H2SO4, and prasugrel.

The pharmaceutical formulations of the invention are preferably provided in a solid unit dosage form, each dosage containing about 1-600 mg of the active substance(s) in its free form. In case the hemifumarate salt of aliskiren is used, a therapeutic dose of 75 mg, 150 mg, 300 mg and 600 mg of free aliskiren corresponds to 82.88 mg, 165.75 mg, 331.50 mg and 663.00 mg of aliskiren hemifumarate, respectively.

The term "unit dosage form" refers to physically discrete units suitable as unitary dosages for humans and other mammals, each containing a predetermined quantity of active ingredient calculated to produce the desired therapeutic effect, in association with a suitable pharmaceutical excipient.

In the process of the present invention, the ratio of the total amount of excipients added in steps (a) and (b) to water is less than 100:30, preferably less than 100:20, in particular less than 100:10, based on weight. In particular, the amount and contacting time of the mixture of the active ingredient(s) and excipients are selected such that a measurable chemical degradation and/or undesired physical phase transition does not occur, although a granulation liquid is used which is known for the respective active substance to have in principle the potential of causing such undesired changes. The concentration of dissolved or dispersed solid components in the granulation liquid, if present, is less than 25 weight%, preferably less than 15 weight%.

The following sections describe the details of the excipients used in the present invention. In general, excipients are used which are required to prepare a stable pharmaceutical dosage form such as in particular a tablet.

More specifically, in accordance with the present invention, diluents can be selected from soluble saccharides such as mannitol, xylitol, powdered cellulose, microcrystalline cellulose, starch and its derivatives, alkaline earth metal phosphates such as calcium hydrogen phosphate in hydrated or anhydrous form.

Surfactants can in general be selected from nonionic or ionic surfactants having an HLB value of more than 8, preferably of more than 10. Nonionic surfactants can in particular be selected from polyoxyethylated glycol monoethers, cetomacrogol, sorbitan esters (Spans) and polysorbates (Tweens), polyoxyethylene-polyoxypropylene copolymers such as poloxameres, sugar esters with fatty acids with 10 to 22 C atoms. Ionic surfactants can in particular be selected from the group of anionic surfactants such as organic sulphonates (RSO₃⁻), sulphates (ROSO₃⁻) e.g. sodium lauryl sulphate CH₃(CH₂)₁₁SO₄⁻Na⁺, potassium laurate CH₃(CH₂)₁₀COO⁻K⁺ or cationic surfactants selected from the group consisting of organic quaternary ammonium halides, R₄N⁺Cl⁻, cetrimide, a mixture consisting of tetradecyl (about 68%), dodecyl (about 22%), and hexadecyltrimethylammonium bromides (about 7%), as well as benzalkonium chloride, a mixture of alkylbenzyldimethylammonium chlorides of the general formula [C₆H₅CH₂N⁺(CH₃)₂R]Cl⁻, where R represents a mixture of alkyls from C₈H₁₇ to C₁₈H₃₇ or ampholytic surfactants selected from sulfobetaines RN⁺(CE₃)₂CH₂CR₂SO₃⁻), or betains N-Dodecyl-N,N-Dimethylbetaine, C₁₂H₂₅N⁺(CH₃)₂CH₂COO⁻ .

Binders can be selected from povidone with a K value of from 7 to 100, copovidone, polyvinyl alcohol, block copolymer of ethylene oxide and vinyl alcohol sold under trade name Kollicoat IR, microcrystalline cellulose, water soluble types of cellulose ethers such as hydroxyethylcellulose, hydroxypropylcellulose, hydroxypropylmethylcellulose, starch, pregelatinised starch, microcrystalline cellulose. Povidone with a K value of from 7 to 100 such as Povidone K25 or K30 is preferred.

Lubricants for use in the present invention are in general selected from metal stearates comprising magnesium, calcium, sodium, aluminium or zinc stearate, talc, hydrogenated castor oil, sodium stearyl fumarate, partial fatty esters of glycerol such as glycerol monostearate, fatty acids such as stearic acid. Preferably, talc or magnesium stearate are used.

Disintegrants are in general selected from the group consisting of crospovidone, starch, pregelatinised starch, sodium starch glycollate, microcrystalline cellulose, carboxymethylcellulose sodium (CMC-Na) or calcium (CMC-Ca), cross-linked CMC-Na, polacrilin potassium, low-substituted hydroxypropylcellulose or mixtures thereof. A particularly preferred disintegrant is crospovidone.

Granulation aids can be selected from substances having large surface area such as colloidal anhydrous silica, which can have a hydrophilic or hydrophobic surface or porous excipients having large surface area such as of more than 10 m²/g, preferably of more than 50 m²/g and even more preferably of more than 100 m²/g such as colloidal anhydrous silica such as Aerosil 200 or colloidal hydrophobic silica such as Aerosil R972, porous silicone dioxide sold by company Fuji Chemical Industry Co-, Ltd. under trade name Sylysia 350 or magnesium aluminometasilicate sold by same company under trade name Neusilin, and types such as UFL2 and US2. Preferably, colloidal silica sold under trade name Aerosil R972 or Aerosil 200D are used.

Buffering/alkalizing agents can be selected from the group of pH modifiers such as moderately acidic or alkaline reacting compounds. When incorporated into the solid dosage form containing aliskiren in the form of salts, they buffer the pH of the solid composition in the range of 6 to 8, preferably 6.5 to 7.5. The pH of the solid composition is measured by dispersing a crushed tablet in 200 ml of purified water.

Specifically, the buffering/alkalizing agents can in general be selected from substances such as sodium or potassium hydroxide, ammonia solution, mono or dibasic alkali and alkaline earth metal phosphates such as disodium hydrogen phosphate in anhydrous or hydrated state, calcium hydrogen phosphate in anhydrous or hydrated state, alkali and alkaline earth metal hydrogen carbonates, earth alkali carbonates and hydroxy carbonates such as magnesium carbonate; heavy alkaline earth oxides such as magnesium oxide, alkali and alkaline earth metal salts of polycarboxylic acids such as citric acid or mixtures thereof. The buffering/alkalizing agents can be added partly of fully either in dissolved form in the granulation liquid or in a solid form such as in powder form to the dry components of the granulate.

Antioxidants can be selected from butylhydroxyanizole (BHA), butylhydroxytoluene (BHT), ascorbic acid, ascorbic acid metal salts, α-tocopherole and its salts, pharmaceutically acceptable substances having kinone moiety in the molecule such as gallic acid, caffeic acid, quercetin, rutin or combination thereof, and the like.

In a preferred embodiment of the process of the present invention, the active ingredient such as aliskiren is mixed with a combination of a granulation aid based on highly dispersed silica such as colloidal anhydrous silica or colloidal hydrophobic silica, a binder based on a povidone such as povidone with a K value of from 7 to 100, and optionally other excipients such as diluents (e.g. manitol, powdered cellulose, xylitol), glidants/lubricants such as talc and optionally part or the whole amount of buffering/alkalizing agents selected from the group of sodium or potassium hydroxide, ammonia solution, mono or dibasic alkali and alkaline earth metal phosphates. All components are present as powders (step (a) of the process of the invention).

After sufficient mixing, the components are contacted with a granulation liquid which contains water, in a ratio as defined hereinabove, and optionally a buffering/alkalizing agents selected from the group of sodium or potassium hydroxide, ammonia solution, mono or dibasic alkali and alkaline earth metal phosphates. Optionally, the granulation liquid further contains a part or all of the binder used in the pharmaceutical formulation.

After granulating the components by using a suitable device such as preferably a fluid-bed or high-shear granulator, further excipients can and preferably are added. Such excipients are preferably selected from the group of diluents, especially low moisture grades of microcrystalline cellulose, highly dispersed silica such as colloidal anhydrous silica, disintegrants such as crospovidone, and lubricants such as magnesium stearate and talc.

If necessary, the further processing of the mixture after the granulation step can include a drying step and a sieving step.

Examples for suitable preferred granulation aids are silica materials such as colloidal anhydrous silica having a hydrophilic or hydrophobic surface and a specific surface area determined by B.E.T. method (adsorption of nitrogen) of more than 10m²/g, preferably of more than 50m²/g and even more preferably in the range of 100 to 600m²/g. Preferred dilluents are cellulose materials such as microcrystalline cellulose, especially low moisture grades. Suitable lubricants are metal stearates such as alkaline earth metal stearates, for example, magnesium stearate.

In a particularly preferred embodiment of the process of the present invention, aliskiren hemifumarate is used as the active ingredient and is processed as follows.

The active ingredient is preferably mixed with a combination of an excipient from the group of granulation aids, in particular Aerosil R972, a povidone binder, in particular povidone K25 or K30, a diluent such as mannitol or powdered cellulose, glidants/lubricants such as talc and optionally part or the whole amount of buffering/alkalizing agents selected from the group of sodium or potassium hydroxide, mono or dibasic alkali and alkaline earth metal phosphates. All components are present as powders (step (a) of the process of the invention).

After sufficient mixing, the components are contacted with the granulation liquid which contains water, in a ratio as defined hereinabove, and optionally a buffering/alkalizing agents selected from the group of sodium or potassium hydroxide, mono or dibasic alkali metal phosphates, in particular dibasic or tribasic sodium phosphate or calcium hydrogen phosphate dihydrate.

After granulating the components using a fluid-bed or high-shear granulator, microcrystalline cellulose, especially low moisture grades and optionally a portion of colloidal anhydrous silica having a hydrophilic or hydrophobic surface, in particular Aerosil 200D, are added. If necessary, i.e. optionally, the mixture is further dried and sieved.

Afterwards, an additional portion of microcrystalline cellulose, especially low moisture grades and optionally colloidal anhydrous silica, disintegrant (especially crospovidone) and finally a lubricant, e.g. a metal stearate, in particular magnesium stearate, are added.

The pharmaceutical formulation of the present invention can be further processed to oral dosage forms such as tablets or capsules, which optionally can be further coated with a functional film coating. Thereby, the dosage forms are provided with the required physical or biopharmaceutical properties such as resistance against dissolution in acidic media (gastro resistant coating), decreased permeability to moisture and other gases such as oxygen, taste or colour masking and/or smoothening of the surface for easier swallowing in case of tablets.

For example, a gastro resistant coating can be based on polymers insoluble at pH below 7.5, preferably below 6.5, most preferably below 6. Further excipients for use in such coatings are selected from plasticizers, antitacking agents, pigments and colorants, and glidants.

Film coating of tablet cores based on polymers such as polyvinyl alcohol such as Opadry AMB or methacrylic acid derivatives such as Eudragit EPO having decreased permeability for moisture and other gases can be used. Film coating can optionally include other pharmaceutically acceptable inactive ingredient selected from plasticizers such as polyethylene glycol, propylene glycol, antitacking agents such as talc, glycerol monostearate, magnesium stearate, stearic acid, pigments such as metal oxides like iron oxides, titanium dioxide and colorants.

Coated tablets of aliskiren hemifumarate obtained according to the present invention tested with the dissolution method described in Example 7 show dissolution results of more than 80% aliskiren hemifumarate dissolved after 30 minutes.

The pharmaceutical formulation of the present invention can be packaged in accordance with procedures and materials known from the state of the art. For example, as packaging material blisters, glass bottles, containers made of polymeric materials with suitable closure systems may be used. Packing can be performed under normal atmosphere or optionally under an atmosphere having a reduced oxygen concentration, preferably in an inert atmosphere or under reduced relative humidity such as 40% RH, preferably 35% RH and most preferably below 30% RH. Reduced oxygen concentration means that the concentration of oxygen in the gas surrounding the solid composition in the primary packaging is below 18 vol/vol%, preferably below 10 vol/vol% and most preferably below 5vol/vol%.

Any pharmaceutical composition may be prepared and stored in a packaging material usually chosen by those of ordinary skill in the art of maintaining stability of active drugs. Decreased moisture permeability of primary packaging material is preferred in order to diminish moisture sorption by aliskiren containing dosage forms to avoid any changes in drug stability. Low moisture permeable primary packaging materials such as aluminium or polychloro-3-fluoroethylene homopolymer/PVC laminate can be used with the thickness in the range 10 to 40 µm in case of Al/Al blisters and 10 to 110 µm in case of Al-polychloro-3-fluoroethylene homopolymer/PVC laminate blisters. Optionally, dosage forms containing aliskiren or its pharmaceutically acceptable salts can be packed into primary packaging with desiccant. Desiccant can be placed inside the packaging unit together with aliskiren dosage units such as tablets and/or in the closure system or can be incorporated into the walls of the primary packaging unit.

According to a preferred embodiment, contact between the pharmaceutical composition and environmental oxygen may be reduced or suppressed by either packaging the pharmaceutical composition under reduced pressure, packaging in an inert gas atmosphere, using a coating affording protection and stability of the pharmaceutical composition to environmental influences, or by using a packaging wherein the contact between the pharmaceutical composition and oxygen is reduced by the means of oxygen absorbers.

An atmosphere with reduced oxygen content or reduced oxygen partial pressure may be obtained by the use of an atmosphere of reduced pressure, e.g. by creating a partial vacuum by means of a suitable pump or by partial freezing or liquefying the atmosphere, by the use of an inert gas atmosphere, wherein as an inert gas nitrogen or argon may be used, or by the use of absorbents. Suitable absorbents may be selected from the group of commercially available absorbents such as humidity-activated oxygen absorbers, ultraviolet-radiation-activated absorbers, radiation-activated absorbers, microwaves-radiation-activated absorbers, absorbers activated by a combination of activation processes or absorbers without necessity of activation. Examples of commercially available absorbers are Ageless^{™} (Mitsubishi Gas Chemical), ATCO (Standa Industry), FreshPax^{™} (Multisorb Technologies), O-Buster^{™} (Hsiao Sung Non-Oxygen Chemical Co), Biotika Oxygen Absorber (Biotika) and the like.

The invention also provides a stabilized package of aliskiren which is provided with a space for trapping and disposal of free oxygen. Moreover, if the active compounds of the present composition are exhibited to a reduced oxygen partial pressure, the formulation is preferably enclosed in a substantially gas exchange non-permeable material and an atmosphere with reduced oxygen partial pressure is contained in the packaging. The substantially gas exchange non-permeable package is preferably selected from the group consisting of an Al/Al blister, an Alpolychloro-3-fluoroethylene homopolymer/PVC laminate blister.

Final packaging can optionally contain dessicant which can be used in blisters made of aluminium foil, incorporated into closure system of glass and/or polymeric containers or added directly into containers.

The following examples further illustrate the present invention.

### Example 1

| **Component** | **Amount [mg/tablet]** |
|---|---|
| Aliskiren hemifumarate (corresponding to 300mg of aliskiren) | 331,50 |
| Colloidal anhydrous hydrophobic silica (Aerosil R 972) | 5,00 |
| Talc | 15,00 |
| Ca hydrogen phosphate, dihydrate | 88,50 |
| Povidone K25 | 40,00 |
| Dibasic sodium phosphate (Na2HPO4) | 4,00 |
| Water | q.s. |
| **Granulate (before drying)** | *484,00* |
| Microcrystalline cellulose (Avicel PH 200 LM) | 219,00 |
| Colloidal anhydrous silica (Aerosil 200 D) | 5,00 |
| Magnesium stearate | 12,00 |
| **Tablet core** | *720,00* |
| Film coating (Opadry PINK OY-S-24900) | 20 |

### Manufacturing procedure:

The active ingredient was mixed with with colloidal anhydrous hydrophobic silica (Aerosil R972), Ca hydrogen phosphate dihydrate, talc and povidone in powder form. Then, the granulation liquid consisting of purified water and disodium hydrogenphosphate (dibasic sodium phosphate) was sprayed onto the powder mixture and granulating/kneading was performed for totally 100 seconds using a Mi-Pro high-shear granulator. Next, colloidal anhydrous silica (Aerosil 200 D), microcrystalline cellulose, (Avicel PH 200LM) were added and mixed with the other components for 60 seconds. After drying and sieving, magnesium stearate was added as lubricant. Finally, the mixture was compacted into tablets and provided with a film-coating.

### Example 2

| **Component** | **Amount [mg/tablet]** |
|---|---|
| Aliskiren hemifumarate (corresponding to 150mg of aliskiren) | 165,75 |
| Colloidal anhydrous hydrophobic silica (Aerosil R 972) | 3,50 |
| Talc | 11,00 |
| Mannitol | 45,85 |
| Povidone K30 | 25,00 |
| Sodium hydroxide | 0,40 |
| Water | q.s. |
| **Granulate (before drying)** | *251,50* |
| Microcrystalline cellulose (Avicel PH 200 LM) | 53,00 |
| Microcrystalline cellulose (Avicel PH 200 LM) | 43,00 |
| Crospovidone | 5,00 |
| Colloidal anhydrous silica (Aerosil 200 D) | 2,50 |
| Castor oil, hydrogenated | 15,00 |
| **Tablet core** | *370,00* |
| Film coating (Opadry PINK OY-S-24900) | 10 |

The active ingredient was mixed with with colloidal anhydrous hydrophobic silica (Aerosil R972), talc, mannitol, povidone and sodium hydroxide in powder form. Then, the granulation liquid consisting of purified water was sprayed onto the powder mixture. Next, microcrystalline cellulose, (Avicel PH 200LM) was added and granulating/kneading was performed for 1 minute using a Mi-Pro high-shear granulator. After drying and sieving, another portion of microcrystalline cellulose, (Avicel PH 200LM), crospovidone and colloidal anhydrous silica (Aerosil 200 D) were added and mixed with the other components. Afterwards castor oil hydrogenated was added as lubricant. Finally, the mixture was compacted into tablets.

### Example 3

| **Component** | **Amount [mg/tablet]** |
|---|---|
| Aliskiren hemifumarate (corresponding to 150mg of aliskiren) | 165,75 |
| Colloidal anhydrous hydrophobic silica (Aerosil R 972) | 3,50 |
| Talc | 11,00 |
| Xylitol | 45,85 |
| Povidone K30 | 25,00 |
| Sodium hydroxide | 0,40 |
| Water | q.s. |
| **Granulate (before drying)** | *251,50* |
| Microcrystalline cellulose (Avicel PH 200 LM) | 53,00 |
| Microcrystalline cellulose (Avicel PH 200 LM) | 43, 00 |
| Crospovidone | 5,00 |
| Colloidal anhydrous silica (Aerosil 200 D) | 2,50 |
| Castor oil, hydrogenated | 15,00 |
| **Tablet core** | *370,00* |

The active ingredient was mixed with with colloidal anhydrous hydrophobic silica (Aerosil R972), talc, xylitol, povidone and sodium hydroxide in powder form. Then, the granulation liquid consisting of purified water was sprayed onto the powder mixture. Next, microcrystalline cellulose, (Avicel PH 200LM) was added and granulating/kneading was performed for 1 minute using a Mi-Pro high-shear granulator. After drying and sieving, another portion of microcrystalline cellulose, (Avicel PH 200LM), crospovidone and colloidal anhydrous silica (Aerosil 200 D) were added and mixed with the other components. Afterwards castor oil hydrogenated was added as lubricant. Finally, the mixture was compacted into tablets.

### Example 4

| **Component** | **Amount [mg/tablet]** |
|---|---|
| Aliskiren hemifumarate (corresponding to 150mg of aliskiren) | 165,75 |
| Colloidal anhydrous hydrophobic silica (Aerosil R 972) | 3,50 |
| Talc | 7,50 |
| Powdered cellulose | 47,35 |
| Povidone K30 | 27,00 |
| Sodium hydroxide | 0,40 |
| Water | q.s. |
| **Granulate (before drying)** | *251,50* |
| Microcrystalline cellulose (Avicel PH 200 LM) | 58,30 |
| Microcrystalline cellulose (Avicel PH 200 LM) | 46,70 |
| Crospovidone | 5,00 |
| Colloidal anhydrous silica (Aerosil 200 D) | 2,50 |
| Magnesium stearate | 6,00 |
| **Tablet core** | *370,00* |

The active ingredient was mixed with with colloidal anhydrous hydrophobic silica (Aerosil R972), talc, powdered cellulose, povidone and sodium hydroxide in powder form. Then, the granulation liquid consisting of purified water was sprayed onto the powder mixture. Next, microcrystalline cellulose, (Avicel PH 200LM) was added and granulating/kneading was performed. After drying and sieving, another portion of microcrystalline cellulose, (Avicel PH 200LM), crospovidone and colloidal anhydrous silica (Aerosil 200 D) were added and mixed with the other components. Afterwards magnesium stearate was added as lubricant. Finally, the mixture was compacted into tablets.

### Example 5

| **Component** | **Amount [mg/tablet]** |
|---|---|
| Aliskiren hemifumarate (corresponding to 150mg of aliskiren) | 165,75 |
| Colloidal anhydrous hydrophobic silica (Aerosil R 972) | 3,50 |
| Talc | 7,50 |
| Powdered cellulose | 47,35 |
| Povidone K30 | 27,00 |
| Sodium hydroxide | 0,40 |
| Water | q.s. |
| **Granulate (before drying)** | *251,50* |
| Microcrystalline cellulose (Avicel PH 200 LM) | 105,00 |
| Crospovidone | 5,00 |
| Colloidal anhydrous silica (Aerosil 200 D) | 2,50 |
| Magnesium stearate | 6,00 |
| **Tablet core** | *370,00* |

The active ingredient was mixed with with colloidal anhydrous hydrophobic silica (Aerosil R972), talc, powdered cellulose and povidone in powder form. Then, the granulation liquid consisting of purified water and sodium hydroxide was sprayed onto the powder mixture in a fluid-bed granulator with top position of spray nozzle.

After drying and sieving microcrystalline cellulose, (Avicel PH 200LM), crospovidone and colloidal anhydrous silica (Aerosil 200 D) were added and mixed with the other components. Afterwards magnesium stearate was added as lubricant. Finally, the mixture was compressed into tablets.

### Example 6

Comparison of stress stability of formulations according to present invention and reference 150 mg tablets (Rasilez batch No.: S0108) - tablets were packed in aluminium blisters and stored 28 days at 50°C (impurities were determined by HPLC chromatography)

| | Max. individual impurity (%) | Total impurities (%) |
|---|---|---|
| reference | 0.18 | 0.72 |
| Example 2 | 0.15 | 0.57 |
| Example 4 | 0.17 | 0.62 |
| Example 5 | 0.13 | 0.51 |

### Example 7

Dissolution profiles comparison of tablet cores of formulations according to present invention are shown in Table 1, Dissolution method: 1,000 ml of 0.1 M HCl solution at 37°C, apparatus 1 (baskets) according to USP (US Pharmacopoeia) (average of six repetitions was used in the table 1).

**Table 1: Comparison of % of aliskiren dissolved from tablet cores prepared according to examples 2-5 of the present invention**

| Time (min) / Batch No. | Ex. 2 (%) | Ex. 3 (%) | Ex.4 (%) | Ex.5 (%) |
|---|---|---|---|---|
| 0 | 0 | 0 | 0 | 0 |
| 5 | 40 | 29 | 28 | 30 |
| 10 | 65 | 55 | 48 | 52 |
| 15 | 87 | 73 | 65 | 71 |
| 20 | 102 | 86 | 82 | 88 |
| 30 | 104 | 96 | 103 | 102 |

## Claims

1. A moisture-activated granulation process for the manufacture of a pharmaceutical dosage form containing at least one moisture-sensitive active substance selected from the group consisting of aliskiren and its pharmaceutically acceptable salts, said process comprising:
a) mixing the active ingredient(s) with one or more dry excipients in solid form, said excipient being selected in particular from the group consisting of diluents, surfactants, binders, lubricants, disintegrators, granulation aids, buffering/alkalizing agents and antioxidants;
b) contacting the mixture with a granulation -liquid containing water and optionally one or more excipients to form a granulate, said excipient being selected in particular from the group consisting of binders, buffering/alkalizing agents, surfactants and antioxidants,
wherein the ratio of the total amount of the excipients of the granulate to water is less than 100:30, based on weight;
c) further processing the mixture including the addition of further dry excipients to obtain the pharmaceutical dosage form, said excipients being in particular selected from the group consisting of diluents, disintegrants, glidants and lubricants.

2. Process according to claim 1, wherein the ratio of the total amount of the excipients used in steps (a) and (b) to water is less than 100:20, in particular less than 100:10, based on weight.

3. Process according to claim 1 or claim 2, wherein the one or more dry excipients mixed with the active ingredient in step (a) is selected from the group of granulation aids, diluents, lubricants, binders and optionally buffering/alkalizing agents.

4. Process according to claim 3, wherein the granulation aid is selected from substances having a surface area of more than 50 m²/g.

5. Process according to claim 3 or 4 wherein the granulation aid is selected from the group consisting of colloidal anhydrous silicas having a hydrophilic or hydrophobic surface and wherein the binder is selected from the group consisting of povidones having a K value of from 7 to 30 and wherein the lubricant is selected from the group consisting of talc and magnesium stearate.

6. Process according to any of the preceding claims, wherein the granulation liquid contains purified water and optionally buffering/alkalizing agents selected from sodium or potassium hydroxide, and dibasic or tribasic sodium phosphate.

7. Process according to claim 6, wherein the granulation liquid additionally contains an excipient selected from the group consisting of binders, antioxidants and surfactants

8. Process according to any of the preceding claims, wherein the active ingredient is aliskiren hemifumarate.

9. Process according to any of the preceding claims, wherein in step (c) an excipient selected from the group consisting of colloidal anhydrous silicas having a hydrophilic or hydrophobic surface and microcrystalline cellulose is added.

10. Process according to any of the preceding claims, wherein the pharmaceutical dosage form obtained is a dosage form suitable for oral delivery.

11. Process according to claim 10, wherein the dosage form is selected from the group consisting of tablets, film coated tablets, pills, lozenges, sachets, soft and hard gelatin capsules.

12. Process according to any of the preceding claims, wherein the active ingredient is selected from the group consisting of lisinopril, ramipril, enalapril, omeprazole, esomeprazole, rabeprazole, pantoprazole, lansoprazole, teophiline, valsartan, rosuvastatin, atorvastatin, fluvastatin, clopidrogel, prasugrel, orlistat, desloratidine, solifenacin, donepezil and the pharmaceutically acceptable salts thereof.

13. Process according to claim 12, wherein the active ingredient is selected from the group consisting of clopidrogel, prasugrel, solifenacin and the pharmaceutically acceptable salts thereof.

14. Pharmaceutical formulation obtainable by the process according to any of the preceding claims.

15. Pharmaceutical formulation according to claim 14 for the treatment of hypertension, congestive heart failure, angina, myocardial infarction, artherosclerosis, diabetic nephropathy, diabetic cardiac myopathy, renal insufficiency, peripheral vascular disease, left ventricular hypertrophy, cognitive dysfunction, stroke, headache and chronic heart failure.
